# EUROPEAN PATENT SPECIFICATION

(11) **EP 1 999 717 B1**
(45) Date of publication and mention of the grant of the patent: **09.06.2010**
(21) Application number: 07735070.0
(22) Date of filing: 09.03.2007
(51) Int. Cl.: G06T 5/00

(54) **SYSTEMS AND METHODS FOR INTERACTIVE DEFINITION OF REGIONS AND VOLUMES OF INTEREST**
SYSTEME UND VERFAHREN ZUR INTERAKTIVEN BESTIMMUNG VON INTERESSENSBEREICHEN UND -VOLUMEN
SYSTÈMES ET PROCÉDÉS POUR DÉFINITION INTERACTIVE DE RÉGIONS ET DE VOLUMES D'INTÉRÊT

(30) Priority: 17.03.2006 US 783524 P
(43) Date of publication of application: 10.12.2008
(73) Proprietor: Koninklijke Philips Electronics N.V., 5621 BA Eindhoven (NL); Philips Intellectual Property & Standards GmbH, 20099 Hamburg (DE)
(72) Inventor: BUSCH, Marc, NL-5621 BA Eindhoven (NL)
(74) Representative: Schouten, Marcus Maria
(86) International application number: PCT/IB2007/050798
(87) International publication number: WO 2007/107907

(56) References cited:
- XINAPSE SYSTEMS: "Region of Interest (ROI) Creation" MANUAL OF THE MEDICAL IMAGE PROCESSING SOFTWARE JIM, [Online] 19 December 2005 (2005-12-19), XP002443467 Retrieved from the Internet: URL:http://web.archive.org/web/20051219043 238/http://www.xinapse.com/Manual/roi_crea te.html> [retrieved on 2007-07-18]
- XINAPSE SYSTEMS: "Region of Interest - Introduction" MANUAL OF THE MEDICAL IMAGE PROCESSING SOFTWARE JIM, [Online] 19 May 2005 (2005-05-19), XP002443468 Retrieved from the Internet: URL:http://web.archive.org/web/20050519094 738/http://www.xinapse.com/Manual/roi_intr o.html> [retrieved on 2007-07-18]
- BAJLA I ET AL: "An alternative method for electrophoretic gel image analysis in the GelMaster software" COMPUTER METHODS AND PROGRAMS IN BIOMEDICINE, ELSEVIER, AMSTERDAM, NL, vol. 77, no. 3, March 2005 (2005-03), pages 209-231, XP004756470 ISSN: 0169-2607
- ZHOU CHUAN ET AL: "Computerized image analysis: Estimation of breast density on mammograms" MEDICAL PHYSICS, AIP, MELVILLE, NY, US, vol. 28, no. 6, June 2001 (2001-06), pages 1056-1069, XP012011480 ISSN: 0094-2405
- ROBB R A ET AL: "ANALYZE: a software system for biomedical image analysis" VISUALIZATION IN BIOMEDICAL COMPUTING, 1990., PROCEEDINGS OF THE FIRST CONFERENCE ON ATLANTA, GA, USA 22-25 MAY 1990, LOS ALAMITOS, CA, USA,IEEE COMPUT. SOC, US, 22 May 1990 (1990-05-22), pages 507-518, XP010019055 ISBN: 0-8186-2039-0

## Description

The present disclosure is directed to systems and methods for facilitating the definition of regions-of-interest and/or volumes-of-interest to delineate coherent areas and, more particularly, to systems and methods that facilitate the definitions of regions or volumes-of-interest, even for noisy image data, in an interactive and rapid manner, both for two dimensional and three dimensional data/systems. The disclosed systems and methods offer numerous advantages over prior art techniques, including, *inter alia*, elimination of the otherwise time-consuming procedure for defining volumes-of-interest as a sequence of regions-of-interest in successive slices.

The definition of regions and/or volumes-of-interest and the delineation of objects-of-interest are important and frequently performed tasks in clinical imaging. For example, clinicians are frequently called upon to identify and/or define volumes-of-interest (VOIs) in radiotherapy planning, the delineation of tumours or lesions to determine their volume, and the like. The tools that are implemented in today's workstations typically allow the clinician to use geometric primitives (e.g., circles, boxes) to define regions-of-interest (ROIs). In addition, conventional workstations generally allow a clinician to employ a "freehand drawing" mode to define a closed region. The definition of VOIs is often realised as a sequential definition of corresponding ROIs in successive image slices. The process of defining VOIs through such successive imaging techniques is cumbersome and time-consuming.

The manual of the medical image processing software JIM from Xinapse System discloses that this image processing software allows for the creation of ROIs in medical images. For example, a rectangular ROI can be manually created by "drawing a rectangle in the medical images". The rectangular ROI, once created, provides corner handles that can be used to stretch the ROI both vertically and horizontally.

Another approach to defining ROIs is image segmentation. In typical image segmentation techniques, the image information is exploited to automatically delineate coherent areas in the image. For example, U.S. Patent No. 5,757,953 to Jang discloses an automated method and system for region decomposition in digital radiographic images.

According to the Jang '953 disclosure, a digital radiographic image is segmented into various regions and a region thereof is further decomposed into sub-regions, wherein digital image data is acquired and subjected to multiple phases of digital imaging processes. Progressively smoothing techniques are employed to generate smoothed regions at multiple scales. The number of connected components at each scale is computed for each smoothed region and a shape spectrum, which is the number of connected components as a function of scale, is constructed to determine the most stable range of scales and the most stable number of sub-regions into which the region is decomposed. Each pixel is classified into sub-regions according to the geometrical relationship of the connected components detected at most stable scales, and a decomposed map is generated to function as multi-valued templates for further image processing of various decomposed sub-regions. However, image segmentation frequently fails to address a clinician's needs, at least in part because it can be ineffective and/or unreliable when image information is noisy, which is almost always the case for emission data (e.g., PET and SPECT applications).

The identification of regions-of-interest is also relevant in other contexts, e.g., digital photography. Thus, U.S. Patent Publication 2004/0095477 to Maki et al. describes an apparatus that includes a region-of-interest recognition unit and a region-of-interest control unit. The region-of-interest recognition unit contains multiple region-of-interest recognition modules for recognizing a region-of-interest based on image data according to various methods. According to the Maki publication, the region-of-interest control unit selects one region-of-interest recognition module from among the region-of-interest recognition modules and sets region-of-interest information based on the noted recognition result. The region-of-interest recognition module may be selected according to an instruction from a user, or a scene type selected by a scene selection switch of an image capture unit. In addition, the region-of-interest control unit may perform operations such as selecting, enlarging, or reducing the region-of-interest recognized by the region-of-interest recognition module, or changing the region-of-interest recognition conditions according to the user's instructions.

Despite efforts to date, a need remains for systems and methods for identifying and/or defining ROIs and/or VOIs that are easier and more intuitive to use. In addition, a need remains for systems and methods for identifying and/or defining ROIs and/or VOIs that offer enhanced effectiveness for noisy images. These and other needs are satisfied by the disclosed systems and methods, as will be apparent from the description which follows, particularly when read in conjunction with the appended figures.

The present disclosure provides advantageous systems and methods for identifying and defining ROIs and VOIs. The disclosed systems and methods are easier and more intuitive in use as compared to conventional imaging systems/techniques. The disclosed systems and methods are also advantageously effective for identifying and defining ROIs and VOIs from images that include substantial amounts of noise, i.e., noisy images. Indeed, the disclosed systems and methods are generally effective in identification/definition of ROIs and VOIs from images where automatic segmentation approaches generally fail.

According to a preferred embodiment of the present disclosure, the identification and/or definition of an ROI/VOI is split into two aspects, namely (i) the identification/definition of a shape and (ii) the identification/definition of a size. Operating with these two distinct aspects, users of the disclosed systems and methods are able to interactively identify/define a ROI and/or a VOI. Of note, in many clinical situations, the shape of a coherent object can be determined/defined by thresholding using a relatively high value, even if the image is noisy. An initial thresholding process generally yields a region/volume definition that is smaller than intended and/or appropriate. However, according to the present disclosure, the thresholded shape may be scaled interactively until the user/clinician is satisfied with the result.

The disclosed system and method is well-suited for a variety of applications and implementations. According to an exemplary implementation, the disclosed system and method are embodied in appropriate programming and/or firmware that is adapted to operate on a processing unit, e.g., a processing unit associated with a clinical imaging workstation. The programming/firmware is generally adapted to interact with imaging software, e.g., through an appropriate application programming interface (API), and to permit interactive manipulation in connection with an image generated through such imaging system, e.g., a proton emission tomography (PET) system, single photon emission computed tomography (SPECT) system, computed tomography (CT) systems, magnetic resonance imaging (MRI) systems, and the like. In use, the disclosed systems and methods facilitate an advantageous graphical user interface (GUI) for manipulating and refining an image, e.g., a clinical image generated though implementation of a conventional imaging system.

Thus, according to exemplary embodiments of the present disclosure, graphical user interface(s) are provided that permit the identification/definition of ROIs/VOIs through on-screen manipulation of the shape and size of a boundary delineation. In exemplary embodiments, the GUI is controlled through manipulation/movement of a mouse, i.e., a device that controls the movement of a cursor or pointer on a display screen. A clinical user of the disclosed system/method is able to define and modify the shape and size of an image boundary through mouse manipulation, e.g., by rolling the mouse along a hard, flat surface. As the mouse is moved, the programming/firmware associated with the disclosed system/method translates such movement to modifications in shape and/or size of the image boundary. Once a desired shape/size is defined, such shape/size may be "locked in" by clicking a button associated with the mouse, as is known in the art.

According to exemplary embodiments of the present disclosure, movement of the mouse (and the associated cursor on the screen) in a first direction, i.e., along a first axis, adjusts the shape of a boundary delineation, whereas movement of the mouse (and the associated cursor on the screen) in a second direction, i.e., along a second axis, adjusts the size of the boundary delineation. For example, "vertical" movement (y-axis) of the mouse/cursor may effect modifications to the shape of the boundary delineation, whereas horizontal movement of the mouse/cursor (x-axis) may effect modifications to the size of the boundary delineation. The programming/firmware associated with the disclosed system/method is advantageously adapted to translate mouse/cursor movements that are not limited to a single axis into their respective horizontal/vertical vector components, thereby permitting a clinician to simultaneously adjust the shape and size of a boundary delineation. For example, movement of the mouse/cursor at a 45o angle relative to an imaginary x-axis and y-axis would be translated into corresponding modifications to both the shape and size of the boundary delineation.

The magnitude of a boundary delineation modification associated with mouse/cursor movement is generally established by the programming and/or firmware and will be apparent to the clinical user on the screen. Modifications to such "scaling" may be implemented by the clinical user, e.g., through a drop-down menu selection, thereby permitting the clinician to adjust the "sensitivity" of the boundary modification based on clinical and/or user needs.

Visual aspects of the graphical user interface (GUI) associated with the disclosed system and method may take a variety of forms. For example, the cursor on the screen may be reflected by a variety of icons/images, such as:
- Arrow → the position and grey value of the pixel under the mouse curser are shown;
- Hand → the image or parts of it can be dragged by click-holding the mouse button;
- Magnifying glass → a magnified presentation of the area around the mouse curser is shown; and
- Dashed box → user can define a rectangular area by click-holding the mouse button.

The disclosed systems and methods may be implemented with one or more of the foregoing mouse/cursor visualizations, as well as alternatives thereto. The disclosed systems and methods offer clinicians with an advantageous tool for quickly, efficiently and intuitively defining/refining ROIs and VOIs in a wide range of clinical applications. While the disclosed systems and methods are widely applicable to clinical imaging modalities, the image delineation/refinement techniques of the present disclosure have particular applicability and benefit for defining and refining images of small objects, e.g., tumours in oncology studies using the radiotracer fluorodeoxyglucose, i.e., FDG studies.

Additional features and benefits associated with the disclosed systems and methods will be apparent from the detailed description which follows.

To assist those of ordinary skill in the art in making and using the disclosed systems and methods, reference is made to the accompanying figures, wherein:
FIG. 1 are a pair of images illustrating delineation of a region of interest in connection with an image that includes noisy data;
FIG. 2 is a schematic illustrating a two-axis technique for adjusting the shape and size of a boundary delineation according to a preferred embodiment of the present disclosure.

The disclosed systems and methods provide an intuitive way to delineate coherent areas and thereby define regions or volumes of interest, even for noisy image data. The disclosed systems and methods are interactive and allow a clinician to achieve desirable results within seconds. The disclosed systems and methods may be used in a wide range of imaging applications, including applications where the image data is two-dimensional as well as three-dimensional. Clinicians are able to avoid and/or eliminate conventional time-consuming procedures for defining VOIs as a sequence of ROIs in successive slices. Indeed, the systems and methods of the present disclosure allow for fast ROI/VOI definition in clinical imaging applications, including applications involving noisy data where automatic segmentation techniques often fail to give acceptable results.

According to the disclosed systems and methods, the definition of a ROI and/or VOI is split into two aspects, namely shape and size. A clinician is able to simultaneously and interactively adjust and/or modify both aspects, i.e., shape and size, through an efficient, on-screen process. In many clinical situations, the shape of a coherent object can be determined by thresholding using a relatively high value, even if the image is noisy. This will result in a region and/or volume definition that is smaller than intended. Therefore, the system and method of the present disclosure permits a system user/clinician to interactively define and scale the boundary delineation until the user is satisfied with the result.

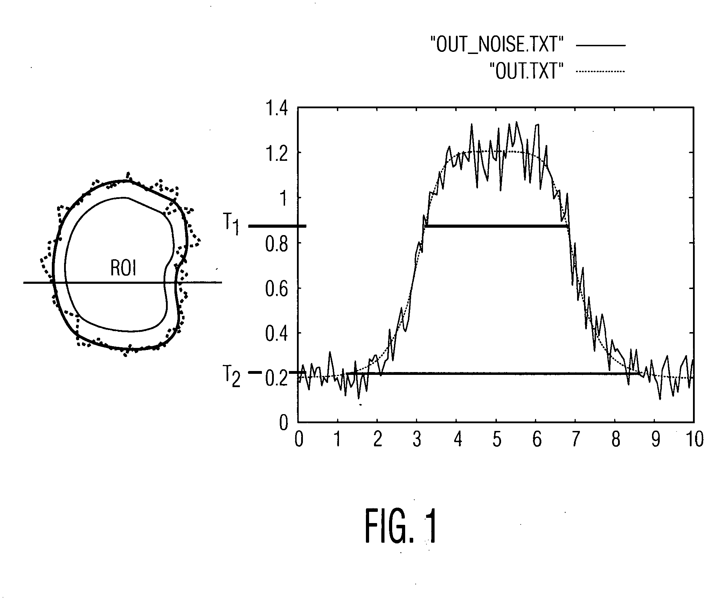

With reference to FIG. 1, imaging data generated according to a conventional clinical imaging technique is set forth. With initial reference to the plot at the right of FIG. 1, it is apparent that the imaging data is noisy (see jagged line that traces a substantial bell curve). For a clinician who desires to delineate the ROI consistent with such jagged line, substantial challenges exist. As the profile plot shows, the jagged region cannot be effectively segmented with a threshold due to the noisy nature of the underlying data. In particular, the threshold T2 would result in an ROI definition as sketched with the dotted line in the left-hand image of FIG. 1. The higher threshold T1 is less sensitive to image noise and therefore reflects the shape of the ROI quite well. However, the defined ROI area in the left-hand image corresponding to the T1 threshold is reflected in the inner-most oval delineation and is smaller than intended or desired.

According to the present disclosure, an advantageous system and method for scaling the ROI (or VOI) delineation boundary is provided. Thus, the present disclosure permits the inner-most boundary delineation of the left-hand image of FIG. 1 to be rapidly, efficiently and intuitively translated to the larger/outer substantially oval boundary delineation that corresponds to the T2 threshold. By increasing the boundary delineation in the manner described herein, the clinician achieves a desired ROI definition despite the exemplary noisy image data reflected in FIG. 1.

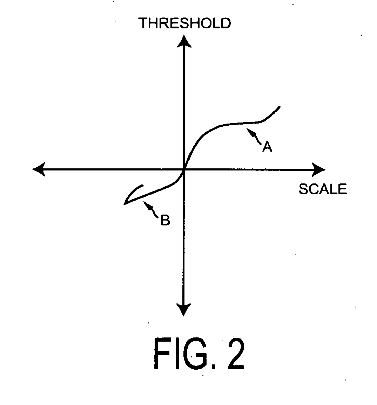

With reference to the schematic of FIG. 2, the systems and methods of the present disclosure permit a system user/clinician to simultaneously adjust/modify two distinct parameters, namely shape and size. Thus, FIG. 2 illustrates a two-axis system wherein the x-axis corresponds to "scale" and the y-axis corresponds to "threshold". Software and/or firmware is provided according to the present disclosure to translate a threshold/scale position on the foregoing axes to a boundary delineation associated with imaging data generated and displayed by a clinical imaging system. Thus, with reference to exemplary Vector A on FIG. 2, the system user/clinician has navigated a path relative to the threshold and scale axes that yields an increased threshold and scale relative to an initial position defined by the intersection of the axes. The path traced by Vector A reflects an exemplary interactive approach to defining a desired boundary delineation wherein the threshold is initially increased at a relatively steep slope and, once a substantially desirable threshold is identified/reached, the scale is adjusted upward. Toward the end of Vector A, the clinician fine tunes both the threshold and scale to arrive at a desired boundary delineation.

A second vector is schematically illustrated on FIG. 2, i.e., Vector B, which reflects an initial reduction in both threshold and scale, followed by a reversal in direction, such that the threshold and scale are increased to arrive at a desired boundary delineation. As with Vector A, exemplary Vector B illustrates the interactive approach to arriving at a desired boundary delineation for an ROI and/or VOI of interest. As will be readily apparent to persons skilled in the art, an infinite number of vectors may be envisioned as a system user/clinician interactively adjusts the threshold and scale of a boundary delineation with respect to imaging data, thereby providing the user/clinician with tremendous flexibility and efficiency in defining such ROIs/VOIs in clinical settings.

The disclosed system and method for boundary delineation of an ROINOI is typically implemented through a mouse-driven graphical user interface (GUI) that interacts and/or communicates with the software for processing imaging data that is generated by a conventional imaging system, e.g., a PET, SPECT or other tomography system. According to exemplary embodiments of the present disclosure, mouse movement is automatically mapped to threshold and scale parameters associated with a boundary delineation. Thus, with reference to FIG. 2, mouse movement corresponding to Vector A will yield a higher threshold and greater scale relative to the starting point (represented by the intersection of the x-axis and the y-axis. In other words, according to the exemplary embodiment schematically depicted in FIG. 2, vertical mouse movement (whether up or down) is automatically translated to a threshold modification, and horizontal mouse movement (whether to the left or to the right) is automatically translated to a scale modification relative, thereby allowing for extremely fast and intuitive ROI/VOI definitions.

Many graphical user interfaces (GUIs) of image processing applications allow selecting specific modes of interaction, which are often reflected by the shape of the mouse curser. Typical examples for these interaction modes are:
- Arrow → the position and grey value of the pixel under the mouse curser are shown;
- Hand → the image or parts of it can be dragged by click-holding the mouse button;
- Magnifying glass → magnified presentation of area around mouse curser shown; and
- Dashed box → user can define a rectangular area by click-holding mouse button.
   Clinical workstations for image interpretation, diagnosis and therapy planning have additional interaction modes that are generally application specific. The systems and methods of the present disclosure can be implemented as one such interaction mode, e.g., similar to the standard ROI definition mode that is part of every such workstation. For illustrative purposes, the disclosed user interface mode could be termed the "shape'n'scale" mode.

Thus, in an exemplary embodiment of the present disclosure, a system user/clinician could select the "shape'n'scale" mode, e.g., from a GUI toolbar or by selecting this mode from a GUI menu. In an exemplary embodiment of the disclosed "shape'n'scale" mode, left clicking and holding the mouse button allows simultaneous variation of two parameters associated with an image boundary delineation. Thus, by moving the mouse in a vertical direction, modifications to the threshold value may be effected, whereas horizontal mouse movement automatically changes the scaling factor. Movements relative to both axes simultaneously and automatically cause changes to both parameters, the magnitude of each such change being predicated on the degree of movement and the associated programming/firmware for implementing the disclosed "shape'n'scale" mode.

An exemplary procedure for defining an ROI and/or VOI according to the present disclosure generally involves the following steps. However, as will be readily apparent to persons skilled in the art, implementation of the disclosed systems and methods is not limited to the disclosed steps and/or the sequence described herein, but rather the disclosed system and method are susceptible to modifications, variations and/or enhancements without departing from the spirit or scope hereof.

The disclosed systems and methods are used in conjunction with a clinical imaging system and, more particularly, with clinical imaging data generated by a clinical imaging system. The clinical imaging data is displayed on a monitor for review by the system user/clinician. Once the system user/clinician has activated and/or selected the disclosed "shape'n'scale" mode, i.e., a system or method that implements the two-aspect delineation control functionalities of the present disclosure, the user/clinician is able to interactively arrive at a desired ROI and/or VOI delineation.

Thus, the user/clinician positions the mouse cursor on the area of interest and activates the "shape'n'scale" functionality, e.g., by click-holding the left mouse button. The value of the pixel at this position (or a fraction of this value) is used as an initial threshold and the coherent area around this position with values above the threshold is shown, e.g., by plotting the contour line of this area. The user/clinician can now decrease/increase the threshold by moving the mouse down/up in vertical direction. Vertical movement of the cursor is automatically translated to a corresponding modification in the threshold for the imaging data through programming/firmware associated with the disclosed system/method. The contour lines are updated in real-time to provide the user with visual feedback as to the impact of his/her threshold modifications. Therefore, finding a parameter setting for the threshold that gives a good representation of the ROI shape is an easy, efficient and intuitive task for the user/clinician. Horizontal movement of the mouse can be used to adapt the scaling factor of the ROI definition. As used herein, scaling is meant as thickening (positive scaling factor) or thinning (negative scaling factor) the area of the defined ROI. Simultaneous vertical and horizontal movement of the mouse cursor effects both modifications to the threshold and scale of the boundary delineation.

If and when the user is satisfied with the boundary delineation result, i.e., the contour line delineates the ROI or VOI as intended, he/she releases the mouse button and the then-present parameter settings for threshold and scale (i.e., shape and size) are used to define the ROI. Such parameter settings (and the associated ROI/VOI) are generally stored and/or utilized in further operations, i.e., in the same manner as ROIs/VOIs that are generated with traditional tools are stored and used. As will be readily apparent to persons skilled in the art, system users and clinicians will be able to adapt both parameters at the same time and intuitively find the intended ROI/VOI in a rapid fashion, e.g., within seconds.

The disclosed systems and methods have wide ranging applicability. For example, the two-aspect approach to ROI/VOI definition may be advantageously utilized as follows:
- Extension to VOI definition for three dimensional images;
- Including algorithms that generate good starting values for the threshold/scale parameters;
- Using the threshold as an *upper* limit to allow ROI definition also for *cold spots;*
- Alternative modes of user interaction, e.g., sliders instead of mouse movement;
- Alternative modes for providing visual feedback, e.g., shaded areas instead of contour lines.

The disclosed systems and methods can be broadly implemented and/or integrated as part of clinical imaging software for several modalities. Thus, the programming/software associated with the disclosed "shape'n'scale" mode may be incorporated into the standard software package for a clinical imaging system, or it may be offered as an optional module that is adapted for operation therewith. In either case, the disclosed systems and methods provide an alternative and improved approach to ROI/VOI boundary delineation, advantageously supporting and/or facilitating interactive ROINOI definition in an efficient and intuitive manner. The disclosed systems and methods may be employed with any clinical imaging data, but is particularly beneficial for modalities like PET, SPECT or low-dose CT, that frequently produce noisy images.

Although the present disclosure describes exemplary embodiments and implementations of the disclosed systems and methods, the present disclosure is not limited to such exemplary embodiments. Rather, the disclosed systems and methods are susceptible to many variations, modifications and/or enhancements within the scope of the appended claims.

## Claims

1. A method for delineating an ROI or VOI, comprising:
- a. providing clinical imaging data;
- b. defining an ROI or VOI with respect to the clinical imaging by simultaneously adjusting two aspects of a boundary delineation for such ROI or VOI;
**characterized in that** the defining of such ROI or VOI comprises thresholding the clinical imaging data to yield a shape of the boundary delineation and scaling the shape to adjust the size of the boundary delineation;
wherein the simultaneous adjustment of the two aspects involves simultaneous adjustment of threshold, which has an influence on the shape, and scale, which has an influence on the size of the boundary delineation.

2. A method according to claim 1, wherein the clinical imaging data is generated by a conventional clinical imaging system, e.g., a PET, SPECT, CT or other tomography system.

3. A method according to any of the preceding claims, wherein the simultaneous adjustment of the two aspects is effected with a mouse.

4. A method according to claim 3, wherein vertical movement of the mouse adjusts one aspect and horizontal movement of the mouse adjusts a second aspect.

5. A method according to claim 4, wherein vertical movement adjusts threshold and horizontal movement adjusts scale.

6. A method according to any of the preceding claims, wherein the step of defining an ROI or VOI is initiated by click-holding a mouse.

7. A method according to any of the preceding claims, further comprising selecting an operation mode from among operation modes, the selected operation mode permitting definition of the ROI or VOI by simultaneous adjustment of two aspects of a boundary delineation.

8. A method according to any of the preceding claims, further comprising storing the defined ROI or VOI.

9. A method according to any of the preceding claims, further comprising utilization of the defined ROI or VOI in further clinical operations.

10. A system for implementing the method of any of the preceding claims.

11. A system according to claim 10, wherein implementation of the method involves operation of software, programming or firmware.

12. A system according to claim 10 or 11, wherein the system is associated with a clinical imaging system.

## Patentansprüche

1. Verfahren zum Abgrenzen eines Interessensbereichs oder Interessensvolumens, das Folgendes umfasst:
- a. Liefern von klinischen Bildgebungsdaten;
- b. Definieren eines Interessensbereichs oder Interessensvolumens in Bezug auf die klinische Bildgebung durch gleichzeitiges Justieren von zwei Aspekten einer Randabgrenzung für einen derartige Interessensbereich oder ein derartiges Interessensvolumen;
**dadurch gekennzeichnet, dass** das Definieren eines derartigen Interessensbereichs oder eines derartiges Interessensvolumens eine Schwellenwertbildung für die klinischen Bildgebungsdaten zum Erlangen einer Form der Randabgrenzung und das Skalieren der Form zum Justieren der Größe der Randabgrenzung umfasst;
wobei die gleichzeitige Justierung der beiden Aspekte das gleichzeitige Justieren des Schwellenwerts, der einen Einfluss auf die Form hat, und des Maßstabs, der einen Einfluss auf die Größe hat, für die Randabgrenzung beinhaltet.

2. Verfahren nach Anspruch 1, wobei die klinischen Bildgebungsdaten durch ein herkömmliches klinisches Bildgebungssystem erzeugt werden, zum Beispiel ein PET-, SPECT-, CT- oder anderes Tomographiesystem.

3. Verfahren nach einem der vorhergehenden Ansprüche, wobei die gleichzeitige Justierung der beiden Aspekte mit einer Maus erfolgt.

4. Verfahren nach Anspruch 3, wobei die vertikale Bewegung der Maus einen Aspekt justiert und die horizontale Bewegung der Maus einen zweiten Aspekt justiert.

5. Verfahren nach Anspruch 4, wobei die vertikale Bewegung den Schwellenwert justiert und die horizontale Bewegung den Maßstab justiert.

6. Verfahren nach einem der vorhergehenden Ansprüche, wobei der Schritt des Definierens eines Interessensbereichs oder eines Interessensvolumens durch Klicken-und-Halten einer Maus initiiert wird.

7. Verfahren nach einem der vorhergehenden Ansprüche, das weiterhin das Auswählen eines Betriebsmodus aus Betriebsmodi umfasst, wobei der ausgewählte Betriebsmodus das Definieren des Interessensbereichs oder des Interessensvolumens durch gleichzeitiges Justieren der beiden Aspekte einer Randabgrenzung erlaubt.

8. Verfahren nach einem der vorhergehenden Ansprüche, das weiterhin das Speichern des definierten Interessensbereichs oder des definierten Interessensvolumens umfasst.

9. Verfahren nach einem der vorhergehenden Ansprüche, das weiterhin die Nutzung des definierten Interessensbereichs und des definierten Interessensvolumens in weiteren klinischen Operationen umfasst.

10. System zum Implementieren des Verfahrens aus einem der vorhergehenden Ansprüche.

11. System nach Anspruch 10, wobei das Implementieren des Verfahrens den Betrieb von Software, Programmierung oder Firmware beinhaltet.

12. System nach Anspruch 10 oder 11, wobei das System zu einem klinischen Bildgebungssystem gehört.

## Revendications

1. Procédé de démarcation d'une région ou d'un volume d'intérêt, comprenant les étapes consistant à :
- a. fournir une donnée d'imagerie clinique ;
- b. définir une région ou un volume d'intérêt par rapport à l'imagerie clinique en réglant simultanément deux aspects d'une démarcation limite pour une telle région ou un tel volume d'intérêt ;
**caractérisé en ce que** l'étape consistant à définir une telle région ou un tel volume d'intérêt comprend l'étape consistant à seuiller la donnée d'imagerie clinique pour obtenir une forme de démarcation limite et réduire la forme pour régler la taille de la démarcation limite ;
le réglage simultané des deux aspects impliquant le réglage simultané du seuillage ayant une influence sur la forme et de la réduction, ayant une influence sur la taille, de la démarcation limite.

2. Procédé selon la revendication 1, la donnée d'imagerie clinique étant générée par un système d'imagerie clinique classique, par exemple, un système PET, TEPU, TDM ou un autre système de tomographie.

3. Procédé selon l'une quelconque des revendications précédentes, le réglage simultané des deux aspects étant réalisé avec une souris.

4. Procédé selon la revendication 3, le déplacement vertical de la souris réglant un aspect et le déplacement horizontal de la souris réglant un second aspect.

5. Procédé selon la revendication 4, le déplacement vertical réglant le seuillage et le déplacement horizontal réglant la réduction.

6. Procédé selon l'une quelconque des revendications précédentes, l'étape consistant à définir une région ou un volume d'intérêt étant déclenchée en cliquant sur la souris et en maintenant la pression.

7. Procédé selon l'une quelconque des revendications précédentes, comprenant en outre l'étape consistant à sélectionner un mode de fonctionnement parmi des modes de fonctionnement, le mode de fonctionnement sélectionné permettant la définition de la région ou du volume d'intérêt en réglant simultanément deux aspects d'une démarcation limite.

8. Procédé selon l'une quelconque des revendications précédentes, comprenant en outre l'étape consistant à enregistrer la région ou le volume d'intérêt défini(e).

9. Procédé selon l'une quelconque des revendications précédentes, comprenant en outre l'étape consistant à utiliser la région ou le volume d'intérêt défini(e) dans d'autres opérations cliniques.

10. Système destiné à mettre en oeuvre le procédé selon l'une quelconque des revendications précédentes.

11. Système selon la revendication 10, la mise en oeuvre du procédé impliquant le fonctionnement du logiciel, de la programmation ou du micrologiciel.

12. Système selon la revendication 10 ou 11, le système étant associé à un système d'imagerie clinique.
